# EUROPEAN PATENT APPLICATION

(11) **EP 3 026 125 A1**
(43) Date of publication of application: **01.06.2016**
(21) Application number: 14194973.5
(22) Date of filing: 26.11.2014
(51) Int. Cl.: C12R 1/01, C12R 1/38, C12R 1/05

(54) **Means and methods for bioremediating glycol ethers**

(71) Applicant: Atotech Deutschland GmbH, 10553 Berlin (DE)
(72) Inventor: Wilde, Christopher, 13509 Berlin (DE)
(74) Representative: Wonnemann, Jörg

(57) **Abstract**

The present invention concerns microorganisms capable of bioremediating glycol ethers, methods of bioremediating glycol ethers utilizing the microorganisms, uses of compositions for bioremediating glycol ether comprising the microorganisms, and screening methods for screening microorganisms capable of bioremediating glycol ethers. The microorganisms and the methods of the present invention can be used for detoxifying process and waste waters in the industry, for example the electronic industry, the paint industry, the chemical industry or the fracking industry.

SEQUENCE LISTING

<110> Atotech Deutschland GmbH

<120> Means and methods for bioremediating glycol ethers

<130> P60445

<160> 6

<170> BiSSAP 1.3

<210> 1
<211> 1526
<212> DNA
<213> Acinetobacter

<220>
<223> 16S rDNA of CW1

<400> 1
gtttgatcct ggctcagatt gaacgctggc ggcaggctta acacatgcaa gtcgagcggg 60

ggaggttgct tcggtaactg acctagcggc ggacgggtga gtaatactta ggaatctgcc 120

tattaatggg ggacaacatc tcgaaaggga tgctaatacc gcatacgccc tacgggggaa 180

agcaggggat cacttgtgac cttgcgttaa tagatgagcc taagtcggat tagctagttg 240

gtggggtaaa ggcctaccaa ggcgacgatc tgtagcgggt ctgagaggat gatccgccac 300

actgggactg agacacggcc cagactccta cgggaggcag cagtggggaa tattggacaa 360

tggggggaac cctgatccag ccatgccgcg tgtgtgaaga aggccttatg gttgtaaagc 420

actttaagcg aggaggaggc tctcttggtt aatacccaag aagagtggac gttactcgca 480

gaataagcac cggctaactc tgtgccagca gccgcggtaa tacagagggt gcgagcgtta 540

atcggattta ctgggcgtaa agcgtgcgta ggcggctttt taagtcggat gtgaaatccc 600

cgagcttaac ttgggaattg cattcgatac tgggaagcta gagtatggga gaggatggta 660

gaattccagg tgtagcggtg aaatgcgtag agatctggag gaataccgat ggcgaaggca 720

gccatctggc ctaatactga cgctgaggta cgaaagcatg gggagcaaac aggattagat 780

accctggtag tccatgccgt aaacgatgtc tactagccgt tggggccttt gaggctttag 840

tggcgcagct aacgcgataa gtagaccgcc tggggagtac ggtcgcaaga ctaaaactca 900

aatgaattga cgggggcccg cacaagcggt ggagcatgtg gtttaattcg atgcaacgcg 960

aagaacctta cctggtcttg acatagtaag aactttccag agatggattg gtgccttcgg 1020

gaacttacat acaggtgctg catggctgtc gtcagctcgt gtcgtgagat gttgggttaa 1080

gtcccgcaac gagcgcaacc cttttcctta tttgccagca cttcgggtgg gaactttaag 1140

gatactgcca gtgacaaact ggaggaaggc ggggacgacg tcaagtcatc atggccctta 1200

cgaccagggc tacacacgtg ctacaatggt cggtacaaag ggttgctacc tagcgatagg 1260

atgctaatct caaaaagccg atcgtagtcc ggattggagt ctgcaactcg actccatgaa 1320

gtcggaatcg ctagtaatcg cggatcagaa tgccgcggtg aatacgttcc cgggccttgt 1380

acacaccgcc cgtcacacca tgggagtttg ttgcaccaga agtaggtagt ctaaccgtaa 1440

ggaggacgct taccacggtg tggccgatga ctggggtgaa gtcgtaacaa ggtagccgta 1500

ggggaacctg cggctggatc acctcc 1526

<210> 2
<211> 1525
<212> DNA
<213> Comamonas

<220>
<223> 16S rDNA of CW2A

<220>
<221> unsure
<222> 521

<400> 2
gagtttgatc ctggctcaga ttgaacgctg gcggcatgct ttacacatgc aagtcgaacg 60

gtaacaggtc ttcggatgct gacgagtggc gaacgggtga gtaatacatc ggaacgtgcc 120

tagtagtggg ggataactac tcgaaagagt agctaatacc gcatgagatc tacggatgaa 180

agcaggggac cttcgggcct tgtgctacta gagcggctga tggcagatta ggtagttggt 240

ggggtaaagg cttaccaagc ctgcgatctg tagctggtct gagaggacga ccagccacac 300

tgggactgag acacggccca gactcctacg ggaggcagca gtggggaatt ttggacaatg 360

ggcgaaagcc tgatccagca atgccgcgtg caggatgaag gccctcgggt tgtaaactgc 420

ttttgtacgg aacgaaaagc ctggggctaa tatccccggg tcatgacggt accgtaagaa 480

taagcaccgg ctaactacgt gccagcagcc gcggtaatac ntagggtgca agcgttaatc 540

ggaattactg ggcgtaaagc gtgcgcaggc ggttttgtaa gacagtggtg aaatccccgg 600

gctcaacctg ggaactgcca ttgtgactgc aaggctagag tgcggcagag ggggatggaa 660

ttccgcgtgt agcagtgaaa tgcgtagata tgcggaggaa caccgatggc gaaggcaatc 720

ccctgggcct gcactgacgc tcatgcacga aagcgtgggg agcaaacagg attagatacc 780

ctggtagtcc acgccctaaa cgatgtcaac tggttgttgg gtcttaactg actcagtaac 840

gaagctaacg cgtgaagttg accgcctggg gagtacggcc gcaaggttga aactcaaagg 900

aattgacggg gacccgcaca agcggtggat gatgtggttt aattcgatgc aacgcgaaaa 960

accttaccca cctttgacat ggcaggaact taccagagat ggtttggtgc tcgaaagaga 1020

acctgcacac aggtgctgca tggctgtcgt cagctcgtgt cgtgagatgt tgggttaagt 1080

cccgcaacga gcgcaaccct tgccattagt tgctacattc agttgagcac tctaatggga 1140

ctgccggtga caaaccggag gaaggtgggg atgacgtcaa gtcctcatgg cccttatagg 1200

tggggctaca cacgtcatac aatggctggt acaaagggtt gccaacccgc gagggggagc 1260

taatcccata aagccagtcg tagtccggat cgcagtctgc aactcgactg cgtgaagtcg 1320

gaatcgctag taatcgtgga tcagaatgtc acggtgaata cgttcccggg tcttgtacac 1380

accgcccgtc acaccatggg agcgggtctc gccagaagta ggtagcctaa ccgcaaggag 1440

ggcgcttacc acggcggggt tcgtgactgg ggtgaagtcg taacaaggta gccgtatcgg 1500

aaggtgcggc tggatcacct ccttt 1525

<210> 3
<211> 1524
<212> DNA
<213> Pseudomonas

<220>
<223> 16S rDNA of CW2C

<400> 3
tttgatcctg gctcagattg aacgctggcg gcaggcctaa cacatgcaag tcgagcggat 60

gaggggagct tgctccctga tttagcggcg gacgggtgag taatgcctag gaatctgcct 120

ggtagtgggg gataacgttc cgaaaggaac gctaataccg catacgtcct acgggagaaa 180

gcaggggacc ttcgggcctt gcgctatcag atgagcctag gtcggattag ctagttggtg 240

aggtaatggc tcaccaaggc gacgatccgt aactggtctg agaggatgat cagtcacact 300

ggaactgaga cacggtccag actcctacgg gaggcagcag tggggaatat tggacaatgg 360

gcgaaagcct gatccagcca tgccgcgtgt gtgaagaagg tcttcggatt gtaaagcact 420

ttaagttggg aggaagggca ttaacctaat acgttagtgt tttgacgtta ccaacagaat 480

aagcaccggc taacttcgtg ccagcagccg cggtaatacg aagggtgcaa gcgttaatcg 540

gaattactgg gcgtaaagcg cgcgtaggtg gttcgttaag ttggatgtga aagccccggg 600

ctcaacctgg gaactgcatc caaaactggc gagctagagt acggtagagg gtggtggaat 660

ttcctgtgta gcggtgaaat gcgtagatat aggaaggaac accagtggcg aaggcgacca 720

cctggactga tactgacact gaggtgcgaa agcgtgggga gcaaacagga ttagataccc 780

tggtagtcca cgccgtaaac gatgtcaact agccgttgga atccttgaga ttttagtggc 840

gcagctaacg cattaagttg accgcctggg gagtacggcc gcaaggttaa aactcaaatg 900

aattgacggg ggcccgcaca agcggtggag catgtggttt aattcgaagc aacgcgaaga 960

accttacctg gccttgacat gctgagaact ttccagagat ggattggtgc cttcgggaac 1020

tcagacacag gtgctgcatg gctgtcgtca gctcgtgtcg tgagatgttg ggttaagtcc 1080

cgtaacgagc gcaacccttg tccttagtta ccagcacctc gggtgggcac tctaaggaga 1140

ctgccggtga caaaccggag gaaggtgggg atgacgtcaa gtcatcatgg cccttacggc 1200

cagggctaca cacgtgctac aatggtcggt acaaagggtt gccaagccgc gaggtggagc 1260

taatcccata aaaccgatcg tagtccggat cgcagtctgc aactcgactg cgtgaagtcg 1320

gaatcgctag taatcgtgaa tcagaatgtc acggtgaata cgttcccggg ccttgtacac 1380

accgcccgtc acaccatggg agtgggttgc tccagaagta gctagtctaa ccttcggggg 1440

gacggttacc acggagtgat tcatgactgg ggtgaagtcg taacaaggta gccgtagggg 1500

aacctgcggc tggatcacct cctt 1524

<210> 4
<211> 1520
<212> DNA
<213> Comamonas

<220>
<223> 16S rDNA of CW3

<400> 4
ttgatcctgg ctcagattga acgctggcgg catgctttac acatgcaagt cgaacggtaa 60

caggtcttcg gatgctgacg agtggcgaac gggtgagtaa tacatcggaa cgtgcctagt 120

agtgggggat aactactcga aagagtagct aataccgcat gagatctacg gatgaaagca 180

ggggaccttc gggccttgtg ctactagagc ggctgatggc agattaggta gttggtgggg 240

taaaggctta ccaagcctgc gatctgtagc tggtctgaga ggacgaccag ccacactggg 300

actgagacac ggcccagact cctacgggag gcagcagtgg ggaattttgg acaatgggcg 360

aaagcctgat ccagcaatgc cgcgtgcagg atgaaggccc tcgggttgta aactgctttt 420

gtacggaacg aaaagcctgg ggctaatatc cccgggtcat gacggtaccg taagaataag 480

caccggctaa ctacgtgcca gcagccgcgg taatacgtag ggtgcaagcg ttaatcggaa 540

ttactgggcg taaagcgtgc gcaggcggtt ttgtaagaca gtggtgaaat ccccgggctc 600

aacctgggaa ctgccattgt gactgcaagg ctagagtgcg gcagaggggg atggaattcc 660

gcgtgtagca gtgaaatgcg tagatatgcg gaggaacacc gatggcgaag gcaatcccct 720

gggcctgcac tgacgctcat gcacgaaagc gtggggagca aacaggatta gataccctgg 780

tagtccacgc cctaaacgat gtcaactggt tgttgggtct taactgactc agtaacgaag 840

ctaacgcgtg aagttgaccg cctggggagt acggccgcaa ggttgaaact caaaggaatt 900

gacggggacc cgcacaagcg gtggatgatg tggtttaatt cgatgcaacg cgaaaaacct 960

tacccacctt tgacatggca ggaacttacc agagatggtt tggtgctcga aagagaacct 1020

gcacacaggt gctgcatggc tgtcgtcagc tcgtgtcgtg agatgttggg ttaagtcccg 1080

caacgagcgc aacccttgcc attagttgct acattcagtt gagcactcta atgggactgc 1140

cggtgacaaa ccggaggaag gtggggatga cgtcaagtcc tcatggccct tataggtggg 1200

gctacacacg tcatacaatg gctggtacaa agggttgcca acccgcgagg gggagctaat 1260

cccataaagc cagtcgtagt ccggatcgca gtctgcaact cgactgcgtg aagtcggaat 1320

cgctagtaat cgtggatcag aatgtcacgg tgaatacgtt cccgggtctt gtacacaccg 1380

cccgtcacac catgggagcg ggtctcgcca gaagtaggta gcctaaccgc aaggagggcg 1440

cttaccacgg cggggttcgt gactggggtg aagtcgtaac aaggtagccg tatcggaagg 1500

tgcggctgga tcacctcctt 1520

<210> 5
<211> 1480
<212> DNA
<213> Pseudochrobactrum

<220>
<223> 16S rDNA of CW4A

<400> 5
ttgagtttga tcctggctca gaacgaacgc tggcggcagg cttaacacat gcaagtcgaa 60

cggtctcttc ggaggcagtg gcagacgggt gagtaatgca tgggaatcta ccgttctcta 120

cggaataact cagggaaact tgtgctaata ccgtatacgc ccttttgggg aaagatttat 180

cggagaatga tgagcccatg ttggattagc tagttggtag ggtaaaggcc taccaaggcg 240

acgatccata gctggtctga gaggatgatc agccacactg ggactgagac acggcccaga 300

ctcctacggg aggcagcagt ggggaatatt ggacaatggg cgcaagcctg atccagccat 360

gccgcgtgag tgatgaaggc cctagggttg taaagctctt tcaccggtga agataatgac 420

ggtaaccgga gaagaagccc cggctaactt cgtgccagca gccgcggtaa tacgaagggg 480

gctagcgttg ttcggattta ctgggcgtaa agcgcacgta ggcggacttt taagtcaggg 540

gtgaaatccc ggggctcaac cccggaactg cctttgatac tggaagtctt gagtatggaa 600

gaggtaagtg gaattgcgag tgtagaggtg aaattcgtag atattcgcag gaacaccagt 660

ggcgaaggcg gcttactggt ccattactga cgctgaggtg cgaaagcgtg gggagcaaac 720

aggattagat accctggtag tccacgccgt aaacgatgaa tgttagccgt cggggtgttt 780

acacttcggt ggcgcagcta acgcattaaa cattccgcct ggggagtacg gtcgcaagat 840

taaaactcaa aggaattgac gggggcccgc acaagcggtg gagcatgtgg tttaattcga 900

agcaacgcgc agaaccttac cagctcttga catgcctatg aatgttagtg gagacacttt 960

cagcctttcg gggcgtagga cacaggtgct gcatggctgt cgtcagctcg tgtcgtgaga 1020

tgttgggtta agtcccgcaa cgagcgcaac cctcgccttt agttgccatc attcagttgg 1080

gcactctaaa gggactgcca gtgataagct ggaggaaggt ggggatgacg tcaagtcctc 1140

atggccctta cgggctgggc tacacacgtg ctacaatggt ggtgacagtg ggcagcgaga 1200

ccgcgaggtc gagctaatct ccaaaagcca tctcagttcg gattgcactc tgcaactcga 1260

gtgcatgaag ttggaatcgc tagtaatcgc ggatcagaat gccgcggtga atacgttccc 1320

gggccttgta cacaccgccc gtcacaccat gggagttggt tttacccgaa ggcgctgtgc 1380

taaccgcaag gaggcaggcg accacggtag ggtcagcgac tggggtgaag tcgtaacaag 1440

gtagccgtag gggaacctgc ggctggatca cctcctttct 1480

<210> 6
<211> 1531
<212> DNA
<213> Alcaligenes

<220>
<223> 16S rDNA of CW4B

<400> 6
tttgatcctg gctcagattg aacgctagcg ggatgcttta cacatgcaag tcgaacggca 60

gcacgagaga gcttgctctc ttggtggcga gtggcggacg ggtgagtaat atatcggaac 120

gtgcccagta gcgggggata actactcgaa agagtggcta ataccgcata cgccctacgg 180

gggaaagggg gggattcttc ggaacctctc actattggag cggccgatat cggattagct 240

agttggtggg gtaaaggctc accaaggcaa cgatccgtag ctggtttgag aggacgacca 300

gccacactgg gactgagaca cggcccagac tcctacggga ggcagcagtg gggaattttg 360

gacaatgggg gaaaccctga tccagccatc ccgcgtgtat gatgaaggcc ttcgggttgt 420

aaagtacttt tggcagagaa gaaaaggtat ctcctaatac gagatactgc tgacggtatc 480

tgcagaataa gcaccggcta actacgtgcc agcagccgcg gtaatacgta gggtgcaagc 540

gttaatcgga attactgggc gtaaagcgtg tgtaggcggt tcggaaagaa agatgtgaaa 600

tcccagggct caaccttgga actgcatttt taactgccga gctagagtat gtcagagggg 660

ggtagaattc cacgtgtagc agtgaaatgc gtagatatgt ggaggaatac cgatggcgaa 720

ggcagccccc tgggataata ctgacgctca gacacgaaag cgtggggagc aaacaggatt 780

agataccctg gtagtccacg ccctaaacga tgtcaactag ctgttggggc cgttaggcct 840

tagtagcgca gctaacgcgt gaagttgacc gcctggggag tacggtcgca agattaaaac 900

tcaaaggaat tgacggggac ccgcacaagc ggtggatgat gtggattaat tcgatgcaac 960

gcgaaaaacc ttacctaccc ttgacatgtc tggaatgccg aagagatttg gcagtgctcg 1020

caagagaacc ggaacacagg tgctgcatgg ctgtcgtcag ctcgtgtcgt gagatgttgg 1080

gttaagtccc gcaacgagcg caacccttgt cattagttgc tacgcaagag cactctaatg 1140

agactgccgg tgacaaaccg gaggaaggtg gggatgacgt caagtcctca tggcccttat 1200

gggtagggct tcacacgtca tacaatggtc gggacagagg gtcgccaacc cgcgaggggg 1260

agccaatctc agaaacccga tcgtagtccg gatcgcagtc tgcaactcga ctgcgtgaag 1320

tcggaatcgc tagtaatcgc ggatcagaat gtcgcggtga atacgttccc gggtcttgta 1380

cacaccgccc gtcacaccat gggagtgggt ttcaccagaa gtaggtagcc taaccgtaag 1440

gagggcgctt accacggtgg gattcatgac tggggtgaag tcgtaacaag gtagccgtat 1500

cggaaggtgc ggctggatca cctcctttct a 1531

## Description

### Field of the Invention

The present invention concerns microorganisms capable of bioremediating glycol ethers, methods of bioremediating glycol ethers utilizing the microorganisms, uses of compositions for bioremediating glycol ether comprising the microorganisms, and screening methods for screening microorganisms capable of bioremediating glycol ethers.

### Background of the Invention

Glycol ethers are widely used in the industry, for example in the paint industry where glycol ethers are constituents of waterborne paints. In the fracking industry of mineral oil companies, glycol ethers are used in fluids which are pumped in the ground to improve the oil or gas recovery. Glycol ethers are also used in degreasing cleaners, in bore- and cutting oil, or in hydraulic fluids. Glycol ethers are also used in the electronics industry, for example in manufacturing of printed circuit boards (PCBs) during cleaning steps in which cleaning solutions containing glycol ethers are employed.

The European production of butyl glycol which is a glycol ether in 1995 was around 90.000 tonnes. Butyl glycol is toxic to animals and microorganisms. A few attempts to dispose of glycol ethers, in particular butyl glycol, can be found in the literature.

The OECD (Organisation for Economic Co-operation and Development) describes butyl glycol as readily biodegradable (OECD Guideline For Testing Of Chemicals, No. 301, 17th July 1992). In low concentrations butyl glycol is stated to be bioremediated by an undefined complex mixture of microorganisms like in activated sludge, communal waste water treatment or soil. In this kind of sludge a broad spectrum of microorganisms is present. In applications in the industry the concentration of butyl glycol is usually much higher and activated sludge or soil is no option for many applications. Further, it was not shown that single microbes can bioremediate glycol ethers.

Patent application JP 2010-130949 A describes the isolation of *rhodococcus* strains that are able of bioremediation of different solvents. Butyl glycol in a low concentration of 200 ppm was almost decomposed within 5 days although the microorganism did not grow.

Patent application US 2006/0122086 A1 discloses compositions and methods for removal of paint overspray in paint spray booths using microorganisms. The removal of paint overspray is obtained by separating an organic phase containing the paint overspray from an aqueous phase by flocculation and coagulation.

Flocculation or coagulation do not result in transformation of toxic or harmful substances into non-toxic or harmless substances. Flocculation or coagulation result in a change of consistence of the substances that facilitates removal from the solution they were contained in and facilitates concentrating the substances. But the substances are still toxic and the flocculated, coagulated or concentrated form has still to be processed to eliminate the toxic or harmful substance in order to protect humans and the environment from its harmful and toxic effects.

Patent application US 2008/0185337 A1 relates to methods for biological degradation of water-borne paint containing high levels of organic solvent and its application for paint detackification and reduction of chemical oxygen demand. Bioremediation of glycol ethers was not shown.

### Objective of the present Invention

It is the objective of the present invention to provide means and methods for degrading glycol ethers. It is a further objective of the present invention to provide means and methods for detoxifying liquids like industrial process waters and waste waters. There is still a need for means and methods for eliminating glycol ethers from industrial process waters and waste waters in an effective, sustainable and low cost way.

### Summary of the Invention

This objective is solved by the microorganism of the present invention, wherein the microorganism is capable of bioremediating glycol ether.

The objective of the present invention is further solved by a method of bioremediating glycol ether, wherein the method comprises treating glycol ether containing material with at least one microorganism of the present invention.

The objective of the present invention is further solved by use of a composition for bioremediating glycol ether comprising at least one microorganism of the present invention.

The objective of the present invention is further solved by a screening method for screening of a microorganism capable of bioremediating glycol ether, said method comprising the steps of:
(a) contacting at least one microorganism with glycol ether containing material; and
(b) measuring
   (i) the amount of glycol ether, and/or
   (ii) the COD;
wherein a reduction in the amount of glycol ether and/or a reduction of the COD is indicative for a microorganism capable of bioremediating glycol ether.

The microorganisms of the present invention are capable of bioremediating glycol ether. They are capable to resist much higher glycol ether concentrations as described so far. The microorganisms of the present invention are applicable to reduce the amount of glycol ethers. Thus, accumulating waste streams of glycol ethers can be detoxified and converted into naturally occurring components like bio mass in a sustainable way.

### Brief Description of the Figures

- Figure 1: illustrates the growth of microorganisms CW1 and CW2A in M458 medium containing butyl glycol as carbon sole source and the resulting COD reduction.
- Figure 2: illustrates the growth of microorganisms CW2C and CW3 in M458 medium containing butyl glycol as carbon sole source and the resulting COD reduction.
- Figure 3: illustrates the growth of microorganisms CW1 and CW2A in M458 medium containing paint overspray water as carbon sole source and the resulting COD reduction.
- Figure 4: illustrates the growth of microorganisms CW2C and CW3 in M458 medium containing paint overspray water as carbon sole source and the resulting COD reduction.

### Detailed Description of the Invention

This invention relates to the bioremediation of glycol ethers, particularly butyl glycol, by microorganisms which have been isolated from chemical waste process waters and its possible industrial applications. Bioremediation, also named biodegradation, is a process performed by microorganisms, in which chemical substances which are exposed to the microorganisms, are converted, utilized and/or incorporated by the microorganisms resulting in the removal of the chemical substance. If the chemical substance is for example a pollution like an oil product, a solvent or a toxic substance bioremediation results in a renaturation or detoxification.

In contrast, coagulation describes the physical-chemical agglomeration of dispersed colloidal particles to form so called colloidal agglomerations. The coagulation process is based on charge rearrangements on the surface of the colloidal particles. The colloidal agglomerations resulting from the coagulation process are still uniformly dispersed throughout the solution or solvent.

Flocculation describes the physical-chemical development of flocs or flakes from colloidal agglomerations. Similar to coagulation the flocculation process is based on charge rearrangements, this time on the surface of the colloidal agglomerations. By accumulating the colloidal agglomerations flocs or flakes form in the size and/or mass to enable sedimentation of the flocs or flakes.

The glycol ether preferably is at least one glycol ether selected from the group consisting of butyl glycol, ethylene glycol, propylene glycol, diethylene glycol monoethyl ether, and butyl diglycol; more preferably butyl glycol.

The microorganism of the present invention is preferably capable of bioremediating butyl glycol.

The microorganism of the present invention more preferably belongs to one genus selected from the group consisting of *Pseudochrobactrum, Comamonas, Alcaligenes, Acinetobacter,* and *Pseudomonas.*

The microorganism of the present invention even more preferably is from a species selected from the group consisting of *Pseudochrobactrum sp., Comamonas sp., Alcaligenes sp., Acinetobacter sp.,* and *Pseudomonas sp* ..

The microorganism of the present invention yet more preferably has a 16S ribosomal RNA gene comprising a DNA sequence containing the base sequence as set forth in any one of SEQ ID NOs. 1 to 6.

The microorganism of the present invention yet more preferably has a 16S ribosomal RNA gene comprising a DNA having an identity of 80% or more, preferably of 90% or more, even more preferably of 95% or more with the base sequence as set forth in any one of SEQ ID NOs. 1 to 6, wherein the microorganism is capable of bioremediating glycol ether.

The microorganism of the present invention yet more preferably is one microorganism selected from the group consisting of
(i) CW1, deposited under the Accession Number DSM 25978;
(ii) CW2A, deposited under the Accession Number DSM 25979;
(iii) CW2C, deposited under the Accession Number DSM 25980;
(iv) CW3, deposited under the Accession Number DSM 25981;
(v) CW4A, deposited under the Accession Number DSM 25982;
(vi) CW4B, deposited under the Accession Number DSM 25983, or
(vii) a variant of a microorganism as described in any one of (i) to (vi), which is capable of bioremediating glycol ether.

Viable samples of the microorganisms of the present invention were deposited in accordance with Rule 31(1)(a) with the DSMZ (Deutsche Sammlung von Mikroorganismen und Zellkulturen GmbH, German Collection of Microorganisms and Cell Cultures, Braunschweig, Germany) which is a depositary institution recognised for the purposes of Rules 31 and 34 EPC. The accession numbers listed above are the accession numbers assigned to each of the microorganisms of the present invention by the DSMZ.

Attempts of identifying the microorganisms of the present invention were performed by the DSMZ (Deutsche Sammlung von Mikroorganismen und Zellkulturen GmbH, German Collection of Microorganisms and Cell Cultures, Braunschweig, Germany). Fatty acids analyses, partial sequencing of 16S rDNA, and collecting physiological data were performed for all microorganisms. Additional phylogenetic and DNA-DNA hybridisation analyses were performed for a selected sub set of microorganisms of the present invention. The results were as follows.

Microorganism CW1 has 95 % similarity to species of the genus *Acinetobacter.* Phylogenetic analysis by complete sequencing of 16S rDNA revealed the highest similarity, 98%, with *Acinetobacter johnsonii.* CW1 is a new species within the genus *Acinetobacter.*

Microorganism CW2A has 100 % similarity to *Comamonas testosteroni.* Phylogenetic analysis by complete sequencing of 16S rDNA revealed the highest similarity, 100 %, with *Comamonas thiooxydans.*

Microorganism CW2C has 99.8 % similarity to *Pseudomonas alcaliphila* and 99.1 % similarity to *Pseudomonas pseudoalcaligenes.* Phylogenetic analysis by complete sequencing of 16S rDNA revealed the highest similarity, 99.9 %, with *Pseudomonas mendocina* and 99.4 % with *Pseudomonas alcaliphila.* DNA-DNA hybridisation analyses between CW2C and *Pseudomonas alcaliphila* (DSM 17744), between CW2C and *Pseudomonas mendocina* (DSM 50071), and between CW2C and *Pseudomonas oleovorans ssp oleovorans* (DSM 1045), revealed that CW2C does not belong to any of the analysed *Pseudomonas* species. CW2C is probably a new species within the genus *Pseudomonas.*

Microorganism CW3 has 100 % similarity to *Comamonas testosteroni.* Phylogenetic analysis by complete sequencing of 16S rDNA confirmed the highest similarity, 99.9 %, with *Comamonas testosteroni.* CW3 might be a strain of *Comamonas testosteroni* or it might be a strain of a new species of the genus *Comamonas.*

Microorganism CW4A has 100% similarity to *Pseudochrobactrum asaccharolyticum.* Phylogenetic analysis by complete sequencing of 16S rDNA revealed the highest similarity, 99.9 %, with *Pseudochrobactrum saccharolyticum,* and 99.9 % with *Pseudochrobactrum lubricantis.* A clear identification of CW4A as one of the *Pseudochrobactrum* species mentioned or as a new *Pseudochrobactrum* species was not possible.

Microorganism CW4B has 99.8% similarity to *Alcaligenes aquatilis.* Phylogenetic analysis by complete sequencing of 16S rDNA confirmed the highest similarity, 99.9 %, with *Alcaligenes aquatilis.* CW4B might be a strain of *Alcaligenes aquatilis* or it might be a new species of the genus *Alcaligenes.*

In summary, most of the microorganisms of the present invention were not known until now. However, according to the analyses of the DSMZ most of the microorganisms of the present invention have high similarity with already known microorganisms. The known microorganisms are called related type strains herein. Some of the related type strains of the DSMZ have been tested for their ability to bio remediate glycol ethers in the same experimental setup as the microorganisms of the present invention. These experiments are described in Example 7 herein. None of the related type strains were able to biodegrade butyl glycol or to utilize butyl glycol as carbon sole source. Regardless of the 16S rDNA sequence of the related type strains having high similarity or being identical, the microorganisms of the present invention still differ in the rest of the genome which includes the information for bioremediating glycol ethers.

The present invention further relates to a method of bioremediating glycol ether, wherein the method comprises treating glycol ether containing material with at least one microorganism of the present invention. The method of the present invention may use one single microorganism strain or a combination of microorganism strains as the at least one microorganism of the present invention.

The method of the present invention, wherein
(A) said glycol ether containing material is treated with said at least one microorganism in a bioreactor;
(B) said at least one microorganism is immobilized on a carrier material, and/or
(C) said at least one microorganism is sprayed on said glycol ether containing material.

The carrier material may be plastics or wood. The glycol ether containing material preferably is a solution. The at least one microorganism preferably is in suspension in this solution.

The present invention further relates to the use of a composition for bioremediating glycol ether. The composition comprises at least one microorganism of the present invention. The composition of the present invention may comprise one single microorganism strain or a combination of microorganism strains as the at least one microorganism of the present invention.

The method of the present invention or the use of the present invention are for bioremediating glycol ether
(viii) in the industry;
(ix) in the household, and/or
(x) to protect the environment.

The method of the present invention or the use of the present invention are for detoxification and/or COD reduction
(xi) of waste water in the electronics industry;
(xii) of waste- and/or process water in the paint industry;
(xiii) of water based systems in the chemical industry;
(xiv) of soil in the fracking industry, and/or
(xv) of material containing degreasing cleaners, bore oil, cutting oil, and/or hydraulic fluid.

Process water is water which is necessary to enable a constant technical process. Process water can circulate and can be used multiple times before transferred to waste disposal or purification. Examples are circulating water of paint spray booths, cooling water, rinsing water.

The COD (chemical oxygen demand) is an indirect measure of the amount of organic compounds in water. The COD of a solution is determined by oxidizing all organic compounds and measuring the amount of oxygen needed. It is expressed in milligrams per liter (mg/L also referred to as ppm (parts per million)), which indicates the mass of oxygen consumed per liter of analysed solution.

An example for industrial application of the microorganisms and methods of the present invention is bioremediation of glycol ethers, particularly butyl glycol, for the purpose of detoxification and COD reduction in waste- or process waters like circulating water in the paint industry where butyl glycol is a constituent of waterborne paints.

Within spray applications of wet paints in spray booths used by the paint industry, the overspray of paint is collected in a water curtain which is connected to a water tank. Depending on the kind of paint, solvent or waterborne paint, different solvents in different quantities are accumulating in this tank.

Especially waterborne paints enrich the water of this tank with solvents, mainly with glycol ethers like butyl glycol, since the evaporation of these solvents during the paint spray application is very low. Due to the accumulation of solvents and other components related to paint, the water has to be changed from time to time. A parameter which has to be determined for the disposal of the water is the Chemical Oxygen Demand (COD). This parameter describes the amount of oxygen, which is necessary to fully oxidize the components in the water and can often be directly related to the main COD contributing glycol ethers in the water. A high COD value increases the disposal costs.

The microorganisms and methods of the present invention can be used to convert butyl glycol into biomass within water based systems of the chemical industry. The difference to former described bioremediation of substances like butyl glycol are the dedicated, single species which are able to permit the process instead of the usage of activated sludge like in biological waste water treatment. Due to hygienic aspects, the application of the microbial species described herein is now reasonable. Modern customers, especially in Europe change more and more from the traditional paint over spray treatment in water to a dry adsorbing technique (Eco Dry Scrubber™ from the company Dürr), which allows to adsorb the paint overspray on adsorber material in an airflow. Even within this technology the microorganisms and methods of the present invention are able to bioremediate butyl glycol to underline the sustainability of this technique.

An example for environmental protection by the microorganisms and methods of the present invention is the fracking industry of mineral oil companies. Glycol ethers, in particular butyl glycol, are used in fluids which are pumped in the ground to improve the oil or gas recovery. Residues of these fluids may stay in the environment. The microorganisms and methods of the present invention are capable to bioremediate i.e. the butyl glycol content in terms of environmental protection.

The microorganisms and methods of the present invention may be generally applied for utilization and/or detoxification of glycol ethers, in particular butyl glycol, by converting this component into biomass. Glycol ethers, in particular butyl glycol, are also used for example in degreasing cleaners, in bore- and cutting oil, or in hydraulic fluids. Furthermore, glycol ethers, in particular butyl glycol, can be used as precursor for further applications, materials or processes.

Glycol ethers are also used in the electronics industry, for example during some manufacturing steps of printed circuit boards (PCBs). In this case some fluids are used in cleaning steps included in the manufacturing process of PCBs which contain butyl glycol or butyl diglycol (so called sweller). The wastewater streams of these production lines contain high levels of butyl glycol as well, which can be detoxified by the microorganisms and methods of the present invention in terms of environmental protection.

The present invention further relates to a screening method for screening of a microorganism capable of bioremediating glycol ether. The method comprises the steps of:
(a) contacting at least one microorganism with glycol ether containing material; and
(b) measuring
   (i) the amount of glycol ether, and/or
   (ii) the COD;
wherein a reduction in the amount of glycol ether and/or a reduction of the COD is indicative for a microorganism capable of bioremediating glycol ether.

The screening method of the present invention may further comprise the step:
(c) comparing the result of (b) with the corresponding result of a corresponding control sample, wherein said control sample has not been contacted with said at least one microorganism.

The microorganisms, or the method of bioremediating glycol ether of the present invention are capable of bioremediating glycol ethers in solutions, wherein the glycol ether concentration is more than 200 ppm, preferably at least 400 ppm. The microorganisms, or the method of bioremediating glycol ether of the present invention are suited for use in bioremediating glycol ethers in solutions, wherein the glycol ether concentration is more than 200 ppm, preferably at least 400 ppm. The screening method of the present invention is capable of screening of a microorganism capable of bioremediating glycol ether in a solution, wherein the glycol ether concentration is more than 200 ppm, preferably at least 400 ppm.

The microorganisms, or the method of bioremediating glycol ether of the present invention are capable of bioremediating glycol ethers, wherein the glycol ether is converted to bio mass. The microorganisms, or the method of bioremediating glycol ether of the present invention are suited for use in bioremediating glycol ethers, wherein the glycol ether is converted to bio mass. The screening method of the present invention is capable of screening of a microorganism capable of bioremediating glycol ethers, wherein the glycol ether is converted to bio mass.

The microorganisms, or the method of bioremediating glycol ether of the present invention are capable of bioremediating glycol ethers, wherein the glycol ether containing material is treated with the microorganism or the composition of the present invention. The microorganisms, or the method of bioremediating glycol ether of the present invention are suited for use in bioremediating glycol ethers, wherein the glycol ether containing material is treated with the microorganism or the composition of the present invention. The screening method of the present invention is capable of screening of a microorganism capable of bioremediating glycol ethers, wherein the glycol ether containing material is treated with the microorganism or the composition of the present invention.

The microorganisms, the method of bioremediating glycol ether, the use, or the screening method of the present invention, wherein the glycol ether is the sole carbon source; wherein preferably the glycol ether containing material is the sole carbon source.

The microorganisms, or the method of bioremediating glycol ether of the present invention are capable of bioremediating glycol ethers, wherein the microorganism proliferates. The microorganisms, or the method of bioremediating glycol ether of the present invention are suited for use in bioremediating glycol ethers, wherein the microorganism proliferates. The screening method of the present invention is capable of screening of a microorganism capable of bioremediating glycol ethers, wherein the microorganism proliferates.

Glycol ethers, like butyl glycol, belong to the class of compounds of xenobiotica, which means that these molecules are made by humans and not existing in our nature. During evolution of the microorganisms of the present invention they could not have been exposed to butyl glycol before. But the microorganisms have in common, that they have the biochemical capability to utilize glycol ethers, in particular butyl glycol, as carbon sole source which enables them to reproduce themselves without the presence of other common C-atom containing sources like sugars, proteins, peptides or aminoacids. These microorganisms are enabled to completely reproduce from butyl glycol as carbon sole source, which means that every carbon atom inside the cell is originally coming from butyl glycol. This means that butyl glycol is converted by the microorganisms for example into nucleic-, amino- or fatty acids. This means that the bacterial microorganisms of the present invention are able to convert toxic substances, namely butyl glycol or other glycol ethers, into non toxic, environmentally friendly, natural occurring compounds like biomolecules.

The Examples described herein are demonstrating the impressive capabilities of the microorganisms of the present invention to utilize xenobiotics like butyl glycol as carbon sole source to survive and to proliferate. The isolated microorganisms are capable to proliferate in untypical, unnatural environments by using carbon sources, which they could have never been exposed to before in nature.

The microorganisms, or the method of bioremediating glycol ether of the present invention are capable of bioremediating glycol ethers, wherein the glycol ether is butyl glycol. The microorganisms, or the method of bioremediating glycol ether of the present invention are suited for use in bioremediating glycol ethers, wherein the glycol ether is butyl glycol. The screening method of the present invention is capable of screening of a microorganism capable of bioremediating glycol ethers, wherein the glycol ether is butyl glycol.

The microorganisms, or the method of bioremediating glycol ether of the present invention are capable of bioremediating glycol ethers, wherein the glycol ether is reduced by at least 400 ppm per day. The microorganisms, or the method of bioremediating glycol ether of the present invention are suited for use in bioremediating glycol ethers, wherein the glycol ether is reduced by at least 400 ppm per day. The screening method of the present invention is capable of screening of a microorganism capable of bioremediating glycol ethers, wherein the glycol ether is reduced by at least 400 ppm per day.

The microorganisms of the present invention are able to reduce the COD by 3 to 31 % during one day. The microorganisms are able to bioremediate between 0.1 to 0.9 ml/l glycol ether, preferably butyl glycol, per day.

The microorganism CW1 is able to reduce the COD of a solution containing glycol ether, preferably butyl glycol, by 3 to 15 % per day. The microorganism CW1 is able to bioremediate 0.1 to 0.5 ml/l glycol ether, preferably butyl glycol, per day.

The microorganism CW2A is able to reduce the COD of a solution containing glycol ether, preferably butyl glycol, by 19 to 31 % per day. The microorganism CW1 is able to bioremediate 0.6 to 0.9 ml/l glycol ether, preferably butyl glycol, per day.

The microorganism CW2C is able to reduce the COD of a solution containing glycol ether, preferably butyl glycol, by 14 to 19 % per day. The microorganism CW1 is able to bioremediate 0.5 to 0.6 ml/l glycol ether, preferably butyl glycol, per day.

The microorganism CW3 is able to reduce the COD of a solution containing glycol ether, preferably butyl glycol, by 13 to 15 % per day. The microorganism CW1 is able to bioremediate 0.3 to 0.5 ml/l glycol ether, preferably butyl glycol, per day.

Preferably, the concentration of glycol ether ranges from 0.4 ml/l to 20 ml/l, more preferably 4 ml/l. Preferably, the microorganisms grow in a minimal medium during bioremediating glycol ether, more preferably in M458 medium. Preferably, the microorganisms grow at a temperature ranging from 20 to 40 °C, more preferably 24 °C. Preferably, the microorganisms are agitated during grow with 100 to 200 rpm, more preferably 125 rpm.

The present invention is further demonstrated by the following non-limiting examples.

### Examples

### 1. Control Experiment

In order to verify, that COD reduction measured is a result of microbiological activities, a control experiment was conducted, in which M458 medium (minimal medium) including 4 ml/l butyl glycol (from Alfa Aesar, Cas. No.: 111-76-2) was treated following the same way as the bioremediation experiments but without the microbiological inoculation. Shake flasks were incubated in a shaking incubator at 24°C, 125 rpm for 32 days.

The COD value was determined by Hach Lange LC1400 Cuvette tests 0-1000 mg O₂/I according to the standard method for measuring chemical oxygen demand in ISO 6060. In the synthetic growth medium of the present experiments glycol ethers are the only organic compound. Therefore the measured COD is a direct measure of the amount of a glycol ether contained in the synthetic growth medium during growth of the microorganisms of the present invention. Decrease of the COD means degradation of a glycol ether.

The COD value was almost constant in this experiment. Thus, no butyl glycol vanished from the medium, for example by evaporation. An evaporation effect could have caused an erroneous COD reduction, which was not due to microbial degradation.

Furthermore the correlation between different concentrations of butyl glycol in deionized water or M458 medium and the associated COD value was established. Butyl glycol concentrations and corresponding COD values are shown in Table 1.

**Table 1: Butyl glycol concentrations and corresponding COD values**

| Butyl glycol [ml/l] | 0 | 2 | 4 | 6 | 8 | 10 |
|---|---|---|---|---|---|---|
| COD in H2O [ml/l] | 0 | 4080 | 8150 | 12610 | 16380 | 20525 |
| COD in M458 [ml/l] | 13 | 4290 | 8530 | 12930 | 16850 | 20775 |

Composition of M458 medium:

| M458 medium: | | Trace Elements Solution (SL-8): | |
|---|---|---|---|
| Na₂HPO₄ | 2.44 g | Na₂-EDTA | 5.20 g |
| KH₂PO₄ | 1.52 g | FeSO₄ x 7 H₂O | 2.00 g |
| (NH₄)₂SO₄ | 4.00 g | ZnSO₄ x 7 H₂O | 150 mg |
| MgSO₄ x 7 H₂O | 0.20 g | MnCl₂ x 4 H₂O | 100 mg |
| CaCl₂ x 2 H₂O | 0.05 g | H₃BO₃ | 62 mg |
| NaCl | 5.00 g | CoCl₂ x 6 H₂O | 190 mg |
| SL-8 | 1.00 ml | CuCl₂ x 2 H₂O | 17 mg |
| Distilled water | 1000 ml | NiCl₂ x 6 H₂O | 24 mg |
| | | Na₂MoO₄ x 2 H₂O | 36 mg |
| | | Distilled water | 1000 ml |

The pH was adjusted to 7.3 and the medium was autoclaved at 121 °C for 15 min.

### 2. Isolation of microorganisms capable of bioremediating glycol ethers

For the isolation of CW1, CW2A, CW2C, CW3, CW4A and CW4B native samples which included these microorganisms were added to M458 medium including 4 ml/l butyl glycol as carbon sole source in shake flask experiments.

The shake flasks were incubated at 24°C for 2 weeks. Once a cultivation was successful, which was indicated by the presence of turbidity, a sample was analyzed by pour plating technique on M458 agar (15 g/l agar in M458 medium) including 4 ml/l butyl glycol as well. Turbidity was measured as optical density at 600 nm (so called OD600) by a UV/Vis photo spectrometer. The grown colonies on the agar plates were observed microscopically. In case different colonies were present, the different colonies were picked and separated on further agar plates. In fact this only occurred in case of CW2 and CW4, which resulted in CW2A and CW2C strains and in CW4A and CW4B strains, respectively, in further experiments.

### 3. Test method for determining bioremediation of glycol ethers - according to invention

To test the ability of the microorganisms isolated as shown in Example 2 to bioremediate glycol ethers, shake flask cultivations with synthetic media (M458 medium) including 4 ml/l butyl glycol as carbon sole source were conducted. The growth was evaluated by measuring OD600 as described in Example 2. A positive growth was concluded as potential bioremediation of butyl glycol. In case of no growth, control experiments were conducted to proof the viability of the tested microorganisms by replacing butyl glycol with standard C-sources like Glucose or Acetate.

For detailed investigation of the bioremediation of glycol ethers, the microorganisms were cultivated in M458 medium including butyl glycol as carbon sole source. The cultivation itself was carried out at 24 °C and by shaking at 125 rpm. The optical densitiy (OD) of the grown cultures was measured at frequent intervals at 600 nm (OD600) and the chemical oxygen demand (COD) was determined at the same time.

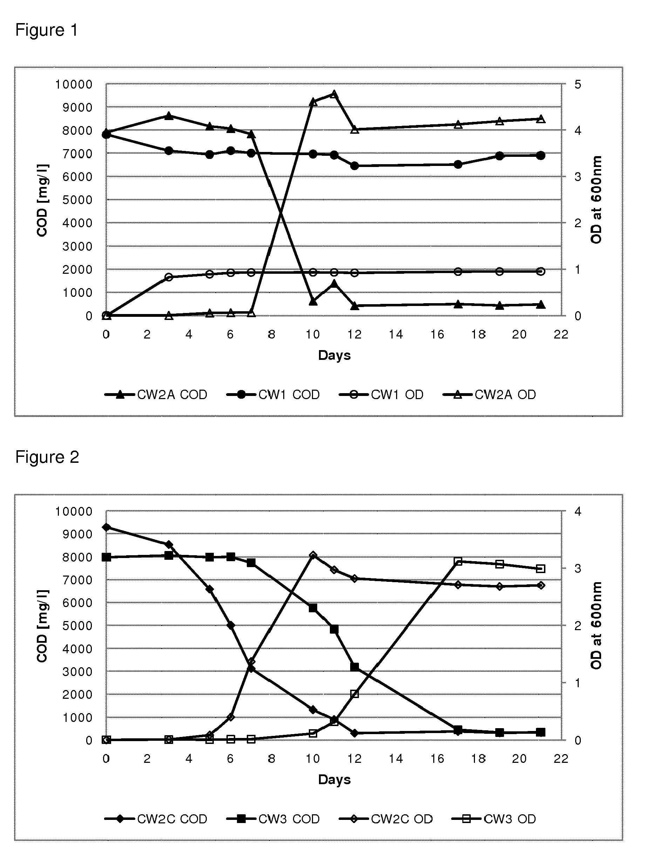

For the exact COD measurement of the grown cultures the presence of biomass (bacterial cells) was considered. When the optical density at 600 nm of the cultivation exceeded 0.5, the biomass was removed from the samples of cultivation solution by filtering with a 0.45 µm polypropylene syringe filter or by centrifuging at 12,800 RCF (relative centrifugal force) for 15 min in order to separate the biomass from the cultivation solution. The COD was measured in the cultivation solution without biomass (COD without biomass). For the purpose of comparison also a sample of cultivation solutions still containing the biomass was measured (COD with biomass). Thus, the contribution of biomass produced by the microorganisms to the COD was eliminated. The resulting COD values only correspond to the concentration of glycol ether still present in the sample. The cultivation experiments were performed twice (Experiment E1 and E2) for each bacterial microorganism. The results are summarized in Tables 2 and 3 and shown in Figures 1 and 2.

**Table 2: Development of OD600 and COD during cultivation of inventive microorganisms**

| | **CW1** | | | **CW2A** | | |
|---|---|---|---|---|---|---|
| Days | OD | COD-[ml/l] | COD + [ml/l] | OD | COD-[ml/l] | COD + [ml/l] |
| 0 | 0.002 | 7800 | 7800 | 0.003 | 7900 | 7900 |
| 3 | 0.828 | 7100 | 7100 | 0.005 | 8620 | 8620 |
| 5 | 0.892 | 6940 | 6940 | 0.053 | 8160 | 8160 |
| 6 | 0.923 | 7100 | 7100 | 0.061 | 8060 | 8060 |
| 7 | 0.929 | 7000 | 7000 | 0.067 | 7820 | 7820 |
| 10 | 0.932 | 6960 | 7420 | 4.61 | 620 | 3080 |
| 11 | 0.93 | 6920 | 6820 | 4.78 | 1390 | 2290 |
| 12 | 0.921 | 6460 | 6920 | 4.01 | 420 | 2180 |
| 17 | 0.947 | 6520 | 7160 | 4.12 | 492 | 2205 |
| 19 | 0.95 | 6880 | 7360 | 4.19 | 432 | 2190 |
| 21 | 0.952 | 6900 | 7280 | 4.24 | 478 | 2160 |

| | **CW2C** | | | **CW3** | | |
|---|---|---|---|---|---|---|
| 0 | 0.002 | 9280 | 9280 | 0.004 | 7980 | 7980 |
| 3 | 0.008 | 8520 | 8520 | 0.008 | 8060 | 8060 |
| 5 | 0.088 | 6580 | 6580 | 0.006 | 7980 | 7980 |
| 6 | 0.404 | 5000 | 5000 | 0.012 | 8000 | 8000 |
| 7 | 1.37 | 3120 | 3120 | 0.016 | 7740 | 7740 |
| 10 | 3.23 | 1320 | 2200 | 0.115 | 5760 | 6780 |
| 11 | 2.97 | 900 | 1720 | 0.321 | 4840 | 5060 |
| 12 | 2.82 | 302 | 1794 | 0.802 | 3180 | 3740 |
| 17 | 2.71 | 376 | 1706 | 3.12 | 450 | 1970 |
| 19 | 2.68 | 316 | 1720 | 3.07 | 330 | 1965 |
| 21 | 2.7 | 331 | 1730 | 2.99 | 337 | 1975 |

| | | | | | | |
|---|---|---|---|---|---|---|
| "OD" = OD600; "COD -" = COD without biomass; "COD +" = COD with biomass | | | | | | |

**Table 3: Bioremediation potential of the microorganisms**

| Microorganisms | CW1 | | CW2A | | CW2C | | CW3 | |
|---|---|---|---|---|---|---|---|---|
| Experiment | E1 | E2 | E1 | E2 | E1 | E2 | E1 | E2 |
| Day of starting growth | 1 | 1 | 7 | 4 | 5 | 5 | 10 | 10 |
| Day of COD maximum | 5 | 2 | 10 | 8 | 12 | 10 | 17 | n.m. |
| Difference of days | 4 | 1 | 3 | 4 | 7 | 5 | 7 | --- |
| COD 0 | 7800 | 7560 | 7900 | 7920 | 9280 | 7880 | 7980 | 7580 |
| COD X | 6853 | 6445 | 639 | 1980 | 331 | 530 | 372 | --- |
| COD reduction [%] | 12 | 15 | 92 | 75 | 96 | 93 | 94 | --- |
| COD reduction [%/day] | 3 | 15 | 31 | 19 | 14 | 19 | 14 | --- |
| Degraded BG [ml/l] | 0.5 | 0.5 | 2.8 | 2.4 | 3.5 | 2.9 | 3.0 | |
| Degraded BG [ml/l day] | 0.1 | 0.5 | 0.9 | 0.6 | 0.5 | 0.6 | 0.4 | |

| | | | | | | | | |
|---|---|---|---|---|---|---|---|---|
| n.m. = not measured; BG = butyl glycol | | | | | | | | |

During the bioremediation of butyl glycol a strong correlation between the increasing OD600 and the decreasing COD could be observed. As soon as the bacteria started to grow, the OD600 increased indicating growth, whereas at the same time the COD was decreased. Since butyl glycol was the only carbon source in the medium, the COD reduction is directly related to the bioremediation of butyl glycol.

In Table 3 the maximum COD reduction in percent was calculated by using the COD value obtained directly after adding microorganisms to the medium (COD 0) and the minimal COD value (COD X). The COD reduction per day was calculated by using the maximum COD reduction and the time difference between the day on which the microorganisms started to grow (Day of starting growth) and the day on which the COD reduction was maximal (Day of COD maximum). Thus, the microorganisms of the present invention are able to reduce the COD by 3 to 31 % during one day. The microorganisms are able to bioremediate between 0.4 to 0.9 ml/l butyl glycol per day.

### 4. Bioremediation of paint overspray water from waterborne paints and its resulting COD reduction

To demonstrate the effectiveness of the microorganisms for the bioremediation of paint solvents and components, dedicated paint overspray water from waterborne paint was mixed with M458 medium in which the overspray water represented the carbon sole source.

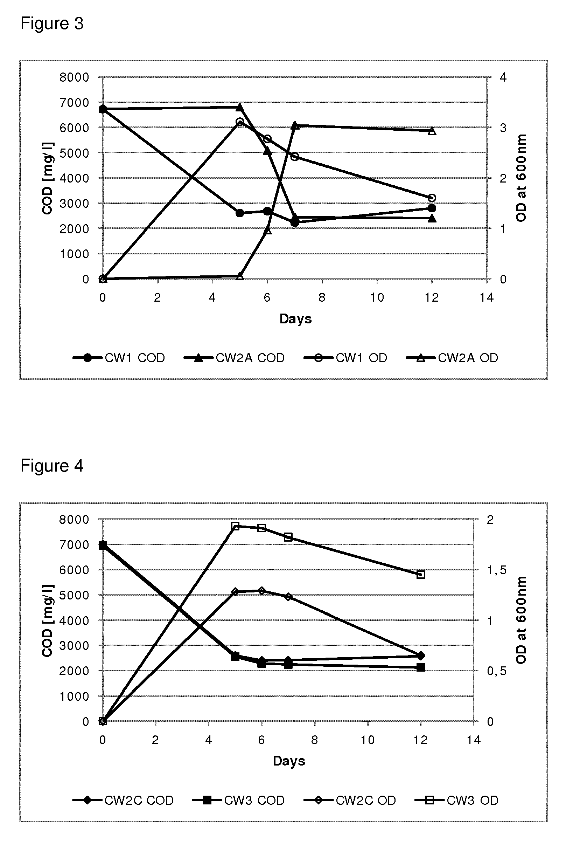

Circulating waters obtained from different painting facilities for waterborne paints were added to M458 medium resulting in approx. 6.5 to 7.0 g/l COD. These mixtures were inoculated by CW1, CW2A, CW2C, CW3 (inventive microorganisms), Bacillus subtilis (comparative) and Pseudomonas fluorescence (comparative) individually. The mixtures were incubated at 24 °C and 125 rpm. The optical density at 600 nm and the COD were measured directly after inoculation (t=0) and hence in frequent intervals of a couple of days. In order to compare the performance of the inventive microorganisms to known microorganisms, two control experiments were conducted, in which Bacillus subtilis and Pseudomonas fluorescence (comparative microorganisms) were tested. The results are summarized in Tables 4 and 5 and shown in Figures 3 and 4.

**Table 4: Development of OD600 and COD during cultivation of inventive microorganisms**

| | CW1 | | CW2A | | CW2C | | CW3 | |
|---|---|---|---|---|---|---|---|---|
| Days | OD | COD-[ml/l] | OD | COD-[ml/l] | OD | COD-[ml/l] | OD | COD-[ml/l] |
| 0 | 0 | 6720 | 0 | 6730 | 0 | 7010 | 0 | 6950 |
| 5 | 3.11 | 2600 | 0.055 | 6800 | 1.28 | 2600 | 1.93 | 2550 |
| 6 | 2.77 | 2675 | 0.969 | 5090 | 1.29 | 2400 | 1.91 | 2280 |
| 7 | 2.42 | 2226 | 3.04 | 2430 | 1.23 | 2410 | 1.82 | 2240 |
| 12 | 1.6 | 2800 | 2.93 | 2400 | 0.65 | 2570 | 1.45 | 2120 |

| | | | | | | | | |
|---|---|---|---|---|---|---|---|---|
| "OD" = OD600; "COD -" = COD without biomass | | | | | | | | |

**Table 5: COD reduction of paint overspray water from water borne paint by the inventive microorganisms**

| Microorganism | COD reduction | Time in days |
|---|---|---|
| CW1 | 66.8 % | 7 |
| CW2A | 64.3 % | 12 |
| CW2C | 65.8 % | 6 |
| CW3 | 69.5 % | 12 |

Neither Bacillus subtilis nor Pseudomonas fluorescence did grow under these conditions and did not reduce the COD. In contrast, all microorganisms according to the invention were able to bioremediate butyl glycol under these conditions which were closer to a real industrial application than in Example 3.

### 5. Enhanced bioremediation of glycol ethers by combinations of microorganisms-according to invention

Furthermore, the combinations of the bacterial microorganisms were tested in order to investigate synergetic effects for the bioremediation of glycol ethers. The combination of CW1 and CW2C (Combi 1), the combination of CW2A and CW2C (Combi 2), the combination of CW1 and CW3 (Combi 3), and the combination of CW1, CW2A, CW2C and CW3 (Combi 4) together were tested in order to bioremediate butyl glycol in M458 medium as described in Example 3. When the OD600 reached 0.5 the samples for the COD measurement were centrifuged to separate the biomass prior to measurement (as described before). The experiment was conducted over a period of 12 days. The results are summarized in Tables 6 and 7.

**Table 6: Development of OD600 and COD during cultivation of combinations of inventive microorganisms**

| | Combi 1 | | Combi 2 | | Combi 3 | | Combi 4 | |
|---|---|---|---|---|---|---|---|---|
| Days | OD | COD-[ml/l] | OD | COD-[ml/l] | OD | COD-[ml/l] | OD | COD-[ml/l] |
| 0 | 0.002 | 7680 | 0.003 | 7860 | 0.003 | 7260 | 0.006 | 7580 |
| 1 | 0.044 | 8520 | 0.004 | 8120 | 0.022 | 8260 | 0.046 | 7880 |
| 4 | 1.36 | 6310 | 0.095 | 7500 | 4.55 | 1150 | 4.99 | 2590 |
| 5 | 1.37 | 6120 | 0.295 | 7130 | 4.66 | 1395 | 4.87 | 2595 |
| 6 | 1.41 | 6150 | 3.97 | 2060 | 4.72 | 1196 | 4.94 | 2372 |
| 7 | 1.39 | 6210 | 4.41 | 2110 | 4.71 | 1272 | 4.98 | 2336 |
| 8 | 1.51 | 6270 | 4.6 | 2250 | 4.87 | 1312 | 4.96 | 2440 |
| 11 | 1.45 | 6210 | 4.56 | 2080 | 4.87 | 1280 | 5.02 | 2408 |
| 12 | 1.41 | 6290 | 4.72 | 2140 | 4.81 | 1292 | 5.04 | 2464 |

| | | | | | | | | |
|---|---|---|---|---|---|---|---|---|
| "OD" = OD600; "COD -" = COD without biomass | | | | | | | | |

**Table 7: Growth peaks of the co-cultivated microorganisms, measured as OD600 during the cultivation. The corresponding COD reduction is given in percentage of the initial COD value (day 0).**

| Combination | Maximal OD | COD reduction | Days |
|---|---|---|---|
| CW1 + CW2C | 1.51 | 20.3 % | 5 |
| CW2A + CW2C | 4.72 | 73.8 % | 6 |
| CW1 + CW3 | 4.87 | 84.2 % | 4 |
| CW1 + CW2A + CW2C + CW3 | 5.02 | 69.2 % | 7 |

All combinations of microorganisms showed high growth and high COD reduction. The COD reduction is improved in comparison to COD reduction of a single microorganism in terms of velocity. Thus, the improved effect or synergistic effect is that the maximum of COD reduction is reached faster or earlier during growth.

### 6. Inhibitory effect of glycol ethers on the growth of the microorganisms - according to the invention

To evaluate whether glycol ethers are toxic to the microorganisms, different concentrations of butyl glycol were added to cultures and the growth of the microorganisms was monitored. The concentration of butyl glycol which is toxic for the microorganisms is relevant for industrial applications, in which butyl glycol is accumulating. When the microorganisms are inhibited or die due to the presence of higher concentration of butyl glycol, the bioremediation of butyl glycol is terminated and hence butyl glycol would increase even more. The influence of butyl glycol to the growth was compared in synthetic minimal medium (M458) and complex medium (ST1). For industrial applications it is of advantage to apply microorganisms which are capable to proliferate under extreme poorness of supplements. The exposition of the microorganisms to butyl glycol under these conditions is even more stressful which was demonstrated in experiments where M458 medium was applied and butyl glycol served as only carbon sole source.

In case of the toxicity test in minimal medium, 5 ml/l, 7.5 ml/l and 10 ml/l butyl glycol were added to M458 which was than inoculated with CW1, CW2A, CW2C or CW3 individually. In case of testing the toxicity in complex medium, 5 ml/l, 10 ml/l or 15 ml/l butyl gycol were added to ST1 media and inoculated with CW1, CW2A, CW2C or CW3 as well. The incubation of the cultures occurred at 24 °C or 30 °C at 125 rpm for up to 37 days. The growth of the microorganisms was evaluated by the measurement of the optical density at 600 nm on regular basis. To differentiate between an inhibiting or toxic effect of butyl glycol to the microorganisms, samples of the cultivations which did not grow inside the medium, were plated on M1 agar (complex medium) without butyl glycol. If growth occurred, the concentration of butyl glycol inside the medium was not toxic, but inhibiting to the growth only. The results are summarized in Tables 8 and 9.

| Composition of complex medium ST1: | | Composition of M1 agar (Medium 1 Nutrient Agar): | |
|---|---|---|---|
| Peptone from soybean | 10 g/l | Peptone | 5g/l |
| Meat Extract | 10 g/l | Meat extract | 3g/l |
| Purified water | | Agar | 15g/l |
| pH | 7.3 | Purified water | |
| | | pH | 7.0 |

The pH was adjusted and the media were autoclaved at 121 °C for 15 min.

**Table 8: Development of OD600 (without biomass) during cultivation of inventive microorganisms in M458 medium**

| | **CW1** | | | **CW2A** | | |
|---|---|---|---|---|---|---|
| Days | Butyl glycol concentration | | | Butyl glycol concentration | | |
| | 5 [ml/l] | 7.5 [ml/l] | 10 [ml/l] | 5 [ml/l] | 7.5 [ml/l] | 10 [ml/l] |
| 0 | 0 | 0 | 0 | 0 | 0 | 0 |
| 2 | 0.115 | 0 | 0 | 0 | 0 | 0 |
| 3 | 0.295 | 0.056 | 0 | 0 | 0 | 0 |
| 6 | 0.326 | 0.114 | 0 | 0 | 0 | 0 |
| 7 | 0.328 | 0.186 | 0 | 0.024 | 0.042 | 0 |
| 8 | 0.334 | 0.303 | 0.016 | 0.03 | 0.042 | 0.148 |
| 9 | 0.335 | 0.575 | 0.016 | 0.145 | 0.048 | 0.353 |
| 13 | 0.336 | 5.33 | 0.018 | 5.02 | 0.159 | 0.45 |
| 17 | 0.34 | 5.41 | 0.018 | 5.04 | 2.11 | 0.429 |
| 20 | 0.345 | 6.47 | 0.018 | 5.57 | 4.06 | 0.456 |
| 24 | 0.344 | 5.38 | 0.015 | 5.06 | 3.88 | 0.722 |
| 30 | 0.349 | 5.77 | 0.021 | 5.26 | 3.88 | 1.848 |
| 37 | 0.346 | 5.44 | 0218 | 5.08 | 3.89 | 1.9 |

| | **CW2C** | | | **CW3** | | |
|---|---|---|---|---|---|---|
| Days | Butyl glycol concentration | | | Butyl glycol concentration | | |
| | 5 [ml/l] | 7.5 [ml/l] | 10 [ml/l] | 5 [ml/l] | 7.5 [ml/l] | 10 [ml/l] |
| 0 | 0 | 0 | 0 | 0 | 0 | 0 |
| 3 | 0 | 0 | 0 | 0 | 0 | 0 |
| 6 | 0.384 | 0 | 0 | 0 | 0 | 0 |
| 7 | 0.997 | 0.042 | 0 | 0.011 | 0.048 | 0 |
| 8 | 1.65 | 0.162 | 0.003 | 0.02 | 0.091 | 0.002 |
| 9 | 2.23 | 0.29 | 0.003 | 0.034 | 0.156 | 0.003 |
| 13 | 2.81 | 5.2 | 0.005 | 0.587 | 1.47 | 0.12 |
| 17 | 2.5 | 5.3 | 0.005 | 3.91 | 2.54 | 0.895 |
| 20 | 2.8 | 5.52 | 0.813 | 3.89 | 2.79 | 2.04 |
| 24 | 2.34 | 5.44 | 0.93 | 3.78 | 2.78 | 2.42 |
| 30 | 2.188 | 5.545 | 0.96 | 3.396 | 2.668 | 2.424 |
| 37 | 2.46 | 5.46 | 0.993 | 3.73 | 2.92 | 2.69 |

**Table 9: Summary of the growth evaluation of the microorganisms when cultivated in ST1 medium**

| Microorganism | Butyl glycol concentration | | |
|---|---|---|---|
| | 5 ml/l | 10 ml/l | 15 ml/l |
| CW1 | Growth | Growth | Growth |
| CW2A | Growth | Growth | Slight growth |
| CW2C | Growth | Growth | No growth |
| CW3 | Growth | Slight growth | No growth |

As becomes apparent from Table 8, the microorganisms also grow in the presence of butyl glycol amounts higher than 4 ml/l (used in the previous Examples) although growth and COD reduction can be decreased (inhibited) at higher concentrations of butyl glycol.

In medium ST1 butyl glycol is not the only carbon sole source (see composition of ST1). The difference to the inhibitory testing of butyl glycol to the growth of the microorganisms in M458 medium is, that other medium components contained in ST1 may stabilize the growth of the microorganisms and make the microorganisms more resistant against a toxic effect of butyl glycol. The stabilizing effect of ST1 components compared to butyl glycol was shown in case of CW1, which did not grow with 10 ml/l butyl glycol in synthetic M458 medium, whereas even 15 ml/l butyl glycol in complex ST1 medium did not inhibit its growth. This knowledge can be used to stabilize microorganisms for industrial applications in order to bioremediate substances which are otherwise too toxic.

### 7. Test of bioremediation of glycol ethers by other microorganisms - comparative

A number of comparative microorganisms was tested for their ability to bioremediate butyl glycol. The intention for these experiments was to compare the microorganisms of the present invention with microorganisms already known to degrade other xenobiotics or with microorganisms having high similarity to the microorganisms of the present invention (related type strains, please see above). The comparative microorganisms were purchased from the DSMZ and were exposed to butyl glycol under the same conditions as described in Example 3. The cultivation was conducted at a temperature and pH value optimal for each individual strain. The results are summarized in Table 10.

**Table 10: Information about comparative microorganisms and their bioremediation of different substances including glycol ethers**

| Microorganism | Known bioremediation of | Bioremediation of butyl glycol |
|---|---|---|
| *Halomonas elongate* (DSM 6909, ATCC 27042) | Ethylene glycol | No |
| *Rhodococcus aetherivorans* (DSM 44752) | Methyl t-butylether | No |
| *Rhodococcus rubber* (DSM 7511, ATCC 51239) | Propane, C5-C16-Alkanes, Benzene, Toluene, Phenol, Benzoate, Naphthalene, Biphenyl | No |
| *Sphingomonas spec.* (DSM 6432) | 4-Chlorodiphenyl ether, Diphenylether, 4-Fluorodiphenylether | No |
| *Geobacillus thermoleovorans* (DSM 5366, ATCC 43513) | n-Alkanes (C13-C20) | No |
| *Torulaspora delbruckii* (DSM 70504) | Isolated from Brandy (resistant to high alcohol concentrations) | No |
| *Alcanivorax borkumensis* | n-Alkanes, methyl ether | No |
| *Acinetobacter bouvetii* (DSM 14964) | Isolated from waste water treatment plant | No |
| *Bacillus subtilis* (DSM 10, ATCC 6051 a) | | No |
| *Pseudomonas fluorescence* (DSM 50090, ATCC 13525) | Acetone, Acrylamide, Benzaldehyde, Ethylbenzene, 1-Propanol, Styrene, o-Xylene | No |
| *Rhodococcus erythropolis* (ATTC 17895) | | No |
| *Comamonas testosteroni* (DSM 50244, ATCC 11996) | | No |
| *Pseudomonas alcaliphila* (DSM 17744) | | No |
| *Phanerochaete chrysosporium* (DSM 6909 and 1556, ATCC 24725 and 34541) | | No |
| *Cunninghamella elegans* (DSM 8217, ATCC 36112) | | No |

| | | |
|---|---|---|
| ATCC = American Type Culture Collection | | |

It turned out, that none of the comparative microorganisms were able to utilize butyl glycol as carbon sole source. This finding demonstrates even more the extraordinary properties of the microorganisms.

### 8. Bioremediation of different glycol ethers

Butyl glycol (BG), ethylene glycol (EG), propylene glycol (PG), diethylene glycol monoethyl ether (DME), and butyl diglycol (BDG) were tested for the possibility of being bioremediated by the inventive microorganisms (CW1, CW2A, CW2C, CW3, CW4A, CW4B) and by comparative microorganisms *(Bacillus subtilis, Pseudomonas fluorescence).* For example, butyl diglycol and ethylene glycol are components of so called sweller solutions used in the electronics industry in the manufacturing process of printed circuit boards. Butyl glycol, for example is contained in waterborne paints. For this purpose M458 medium including either 4 ml/l of the solvents was prepared and inoculated. The cultivation occurred at 24 °C and 125 rpm up to 64 and 91 days to evaluate the growth and the potential of the microorganisms to bioremediate these substances. The growth was determinded by the measurement of the optical density at 600 nm. The results are summarized in Table 11.

**Table 11: Bioremediation of 4 ml/l of glycol ethers in M458 medium.**

| Microorganism | BG (OD600) | EG (OD600) | PG (OD600) | DME (OD600) | BDG [% bioremediation] |
|---|---|---|---|---|---|
| CW1 | 1.32 | No growth | 2.980 | 3.380 | 67.9 |
| CW2A | 4.92 | No growth | No growth | No growth | 85.7 |
| CW2C | 3.23 | 1.453 | 1.450 | 1.850 | 91.1 |
| CW3 | 4.49 | 1.623 | 2.190 | No growth | 85.7 |
| CW4A | turbid | No growth | No growth | Not tested | Not tested |
| CW4B | turbid | 0.342 | 2.660 | Not tested | Not tested |
| *B.* s. | No growth | No growth | No growth | Not tested | Not tested |
| *P. f.* | No growth | No growth | No growth | Not tested | Not tested |

| | | | | | |
|---|---|---|---|---|---|
| *B. s.* = *Bacillus subtilis; P. f.* = *Pseudomonas fluorescence* | | | | | |

All glycol ethers were bioremediated by the microorganisms of the present invention. None of the glycol ethers showed toxic effects on the inventive microorganisms in the used concentration. *Bacillus subtilis* and *Pseudomonas fluorescence* did not bioremediate any of the glycol ethers.

## Claims

1. A microorganism capable of bioremediating glycol ether.

2. The microorganism of claim 1 wherein the glycol ether is at least one glycol ether selected from the group consisting of butyl glycol, ethylene glycol, propylene glycol, diethylene glycol monoethyl ether, and butyl diglycol.

3. The microorganism of claim 1 or 2, wherein the microorganism is capable of bioremediating butyl glycol.

4. The microorganism of any one of claims 1 to 3, wherein the microorganism belongs to one genus selected from the group consisting of *Pseudochrobactrum, Comamonas, Alcaligenes, Acinetobacter,* and *Pseudomonas.*

5. The microorganism of any one of claims 1 to 4, wherein the microorganism has a 16S ribosomal RNA gene comprising a DNA sequence containing the base sequence as set forth in any one of SEQ ID NOs. 1 to 6.

6. The microorganism of any one of claims 1 to 5, wherein the microorganism is one microorganism selected from the group consisting of
(i) CW1, deposited under the Accession Number DSM 25978;
(ii) CW2A, deposited under the Accession Number DSM 25979;
(iii) CW2C, deposited under the Accession Number DSM 25980;
(iv) CW3, deposited under the Accession Number DSM 25981;
(v) CW4A, deposited under the Accession Number DSM 25982;
(vi) CW4B, deposited under the Accession Number DSM 25983, or
(vii) a variant of a microorganism as described in any one of (i) to (vi), which is capable of bioremediating glycol ether.

7. A method of bioremediating glycol ether, wherein the method comprises treating glycol ether containing material with at least one microorganism as described in any one of claims 1 to 6.

8. The method of claim 7, wherein one single microorganism strain or wherein a combination of microorganism strains is used.

9. The method of claim 8, wherein the glycol ether containing material is a solution, and wherein the at least one microorganism is in suspension in this solution.

10. Use of a composition for bioremediating glycol ether comprising at least one microorganism as described in any one of claims 1 to 6.

11. The method of any one of claims 7 to 9, or the use of claim 10, wherein the method or the use is for bioremediating glycol ether
(viii) in the industry;
(ix) in the household, and/or
(x) to protect the environment.

12. The method or the use of claim 11, wherein the method or the use is for detoxification and/or COD reduction
(xi) of waste water in the electronic industry;
(xii) of waste- and/or process water in the paint industry;
(xiii) of water based systems in the chemical industry;
(xiv) of soil in the fracking industry, and/or
(xv) of material containing degreasing cleaners, bore oil, cutting oil, and/or hydraulic fluid.

13. A screening method for screening of a microorganism capable of bioremediating glycol ether, said method comprises the steps of:
(a) contacting at least one microorganism with glycol ether containing material; and
(b) measuring
(i) the amount of glycol ether, and/or
(ii) the COD;
wherein a reduction in the amount of glycol ether and/or a reduction of the COD is indicative for a microorganism capable of bioremediating glycol ether.

14. The microorganism of any one of claims 1 to 6, the method of bioremediating glycol ether of any one of claims 7 to 9, 11, and 12, the use of any one of claims 10 to 12, or the screening method of claim 13, wherein the glycol ether is converted to bio mass.

15. The microorganism of any one of claims 1 to 6, the method of bioremediating glycol ether of any one of claims 7 to 9, 11, and 12, the use of any one of claims 10 to 12, or the screening method of claim 13, wherein the glycol ether is reduced by at least 400 ppm per day.
